# EUROPÄISCHE PATENTANMELDUNG

(11) **EP 2 505 154 A1**
(43) Veröffentlichungstag der Anmeldung: **03.10.2012**
(21) Anmeldenummer: 11160692.7
(22) Anmeldetag: 31.03.2011
(51) Int. Cl.: A61B 17/70

(54) **Wirbelsäulenimplantat**

(71) Anmelder: Spinelab AG, 8406 Winterthur (CH)
(72) Erfinder: Clark, Jonathan, 8302, Kloten (CH); Braunschweiler, Reto, 8413, Neftenbach (CH); Zehnder, Thomas, 8806, Bäch (CH)
(74) Vertreter: BOVARD AG

(57) **Zusammenfassung**

Ein Wirbelsäulenimplantat zur Stabilisierung und Versteifung von Wirbelkörpern einer Wirbelsäule umfasst mindestens eine erste Knochenschraube (3), welche in einen Wirbelkörper einschraubbar ist, und mindestens eine zweite Knochenschraube (10), welche ebenfalls in einen Wirbelkörper einschraubbar ist. Ein erstes Verbindungselement, das starr ist, ist in erste Aufnahmemittel (5) der ersten Knochenschraube (3) einsetzbar. Ein zweites Verbindungselement (9), das elastisch ist, ist in zweite Aufnahmemittel (13) der zweiten Knochenschraube einsetzbar. Über Verbindungsmittel (16) wird das erste Verbindungselement (2) mit dem zweiten Verbindungselement (9) verbunden. Die Verbindungsmittel (16) sind an einer weiteren Knochenschraube (18) angebracht und bestehen aus einer ersten Spanneinrichtung (20) zum Festhalten des ersten Verbindungselementes (2) und aus einer zweiten Spaneinrichtung (21) zum Festhalten des zweiten Verbindungselementes (9). Die erste Spanneinrichtung (20) weist eine U-förmige Aufnahme (22) für den Endbereich (14) des ersten Verbindungselementes auf, der kugelförmig ausgebildet ist, wobei auf die U-förmige Aufnahme (22) ein Spannmittel aufsetzbar ist, mit welchem der kugelförmige Endbereich (14) gegen die Grundfläche (23) der U-förmigen Aufnahme (22) spannbar ist.

## Beschreibung

Die vorliegende Erfindung bezieht sich auf ein Wirbelsäulenimplantat zur Stabilisierung und Versteifung von Wirbelkörpern einer Wirbelsäule, umfassend mindestens eine erste Knochenschraube, bestehend aus einem Einschraubteil, welcher in einen Wirbelkörper einschraubbar ist und ersten Aufnahmemitteln, mindestens eine zweite Knochenschraube, bestehend aus einem Einschraubteil, welcher in einen Wirbelkörper einschraubbar ist, und zweiten Aufnahmemitteln, mindestens ein erstes Verbindungselement, das starr ist und in die ersten Aufnahmemittel der ersten Knochenschraube einsetzbar und darin befestigbar ist, mindestens ein zweites Verbindungselement, das elastisch ist und in die zweiten Aufnahmemittel der zweiten Knochenschraube einsetzbar und darin befestigbar ist, und Verbindungsmittel, mit welchen jeweils ein erstes Verbindungselement mit einem zweiten Verbindungselement verbindbar ist.

Derartige Wirbelsäulenimplantate zur Stabilisierung und Versteifung von Wirbelkörpern einer Wirbelsäule sind in vielfältiger Weise bekannt. Mit dem versteifenden Teil dieses Wirbelsäulenimplantats wird erreicht, dass zwischen den derartig versteiften Wirbelkörpern ein knöchernes Verwachsen stattfindet, was zu einer gewünschten Stabilisierung der Wirbelsäule führt, während mit dem elastischen Bereich des Wirbelsäulenimplantats die Wirbelkörper stützend stabilisiert werden. Eine gewisse Beweglichkeit zwischen den einzelnen Wirbelkörpern ist erwünscht und wird zugelassen.

Ein derartiges Wirbelsäulenimplantat zur Stabilisierung und Versteifung von Wirbelkörpern einer Wirbelsäule ist beispielsweise aus der EP-A-1 961 392 bekannt.

Bei diesen bekannten Wirbelsäulenimplantaten muss das Setzen der Knochenschrauben in den einzelnen Wirbelkörpern insbesondere im Bereich des Übergangs vom elastischen Verbindungselement zum starren Verbindungselement sehr genau erfolgen. Gewisse Ungenauigkeiten können dadurch auskorrigiert werden, indem das starre Verbindungselement passend gebogen wird, was während des Einsetzens des Wirbelsäulenimplantats in eine Wirbelsäule ausgeführt werden muss und den Eingriff erschwert.

Die Aufgabe der vorliegenden Erfindung besteht somit darin, ein Wirbelsäulenimplantat zur Stabilisierung und Versteifung von Wirbelkörpern einer Wirbelsäule zu schaffen, welches einfach im Aufbau ist und dadurch das Einsetzen und Verbinden der starren und elastischen Verbindungselemente vereinfacht wird, und bei welchem insbesondere das erste Verbindungselement, das starr ist, bzgl. dem zweiten elastischen Verbindungselement allseitig in einem gewissen Winkelbereich ausgerichtet werden kann.

Erfindungsgemäss erfolgt die Lösung dieser Aufgabe dadurch, dass die Verbindungsmittel an einer weiteren Knochenschraube angebracht sind und aus einer ersten Spanneinrichtung zum Festhalten des ersten Verbindungselements und aus einer zweiten Spanneinrichtung zum Festhalten des zweiten Verbindungselements bestehen, und dass die erste Spanneinrichtung eine U-förmige Aufnahme für den Endbereich des ersten Verbindungselementes aufweist, welcher Endbereich kugelförmig ausgebildet ist, und auf die U-förmige Aufnahme ein Spannmittel aufsetzbar ist, mit welchem der kugelförmige Endbereich gegen die Grundfläche der U-förmigen Aufnahme spannbar ist.

Durch diese Ausgestaltung kann das erste starre Verbindungselement bzgl. der ersten Spanneinrichtung und somit bzgl. dem zweiten elastischen Verbindungselement allseitig in einem gewissen Winkelbereich ausgerichtet werden und in optimaler an die in die Wirbelkörper eingesetzten Knochenschrauben angepasst werden. Dadurch wird erreicht, dass ein nicht exaktes Setzen der Knochenschrauben in die Wirbelkörper statt durch aufwendiges Verbiegen des starren Verbindungselements während des Eingriffs durch die Möglichkeit einer polyaxialen Ausrichtung des ersten Verbindungselements ausgeglichen werden kann.

In vorteilhafter Weise weist die Grundfläche der U-förmigen Aufnahme eine konkave Kugelform auf, die dem kugelförmigen Endbereich des ersten Verbindungselements entspricht, wodurch eine optimale Pressung und demzufolge Fixierung des kugelförmigen Endbereichs des ersten Verbindungselements in der ersten Spanneinrichtung erhalten wird.

In vorteilhafter Weise weist auch die den kugelförmigen Endbereich des ersten Verbindungselements zugewandte Oberfläche des Spannmittels eine konkave kugelförmige Ausnehmung auf, wodurch das Festhalten und Fixieren des kugelförmigen Endbereichs in der ersten Spanneinrichtung zusätzlich verbessert wird.

In vorteilhafter Weise umfasst das Spannmittel eine Spannschraube, welche in ein in der U-förmigen Aufnahme angebrachtes Gewinde einschraubbar ist, was einen einfachen Aufbau des Spannmittels ergibt.

In vorteilhafter Weise umfasst das Spannmittel ein Presselement, welches zwischen die Spannschraube und den kugelförmigen Endbereich des ersten Verbindungselements einsetzbar ist. Dadurch wird erreicht, dass die Spannschraube nicht in direkten Kontakt mit dem kugelförmigen Endbereich des ersten Verbindungselements kommt, was einer Beschädigung dieses kugelförmigen Endbereichs beim Spannvorgang vorbeugt und somit keine Späne gebildet werden.

In vorteilhafter Weise ist die Spannschraube drehbar mit dem Presselement verbunden, wodurch erreicht wird, dass die Spannschraube und das Presselement als Einheit in die U-förmige Aufnahme eingesetzt werden können, was diesen Einsetzvorgang vereinfacht.

Eine weitere vorteilhafte Ausgestaltung der Erfindung besteht darin, dass die zweite Spanneinrichtung eine weitere U-förmige Aufnahme für den Endbereich des zweiten Verbindungselements aufweist, auf welche ein weiteres Spannmittel aufsetzbar ist, mit welchem der Endbereich des zweiten Verbindungselements in der weiteren U-förmigen Aufnahme gehalten ist. Dadurch können die beiden Verbindungselemente unabhängig voneinander in die Verbindungsmittel eingesetzt und gespannt werden.

In vorteilhafter Weise sind die dem Endbereich des zweiten Verbindungselements zugewandte Fläche des weiteren Spannmittels und/oder die Grundfläche der weiteren U-förmigen Aufnahme mit Strukturen versehen. Dadurch wird eine verbesserte Verbindung zwischen dem zweiten elastischen Verbindungselement und dem weiteren Spannmittel und/oder der Grundfläche der weiteren U-förmigen Aufnahme erhalten.

In vorteilhafter Weise ist mindestens der Endbereich des zweiten Verbindungselements mit weiteren Strukturen versehen, die zu den Strukturen der weiteren U-förmigen Aufnahme komplementär ausgebildet sind, wodurch zwischen dem zweiten Verbindungselement und der weiteren U-förmigen Ausnehmung eine formschlüssige Verbindung erhalten wird.

In vorteilhafter Weise bestehen die Strukturen und die weiteren Strukturen aus Rippen und Rillen, die im Wesentlichen quer zur Längsachse des zweiten Verbindungselements ausgerichtet sind, welche Rippen und Rillen einfach hergestellt werden können.

Eine Ausgestaltung der Erfindung wird nachfolgend anhand der beiliegenden Zeichnungen beispielhaft näher erläutert.

Es zeigt
Fig. 1 in räumlicher Darstellung ein Wirbelsäulenimplantat, das ein erstes starres Verbindungselement, ein zweites elastisches Verbindungselement und Knochenschrauben, in welche die Verbindungselemente eingesetzt und gehalten sind, und eine weitere Knochenschraube umfasst, in welche die Endbereiche der zwei Verbindungselemente eingesetzt und fixiert sind;
Fig. 2 in räumlicher Darstellung die in der weiteren Knochenschraube angebrachte zweite Spanneinrichtung zum Festhalten des zweiten Verbindungselements; und
Fig. 3 eine vergrösserte Schnittdarstellung des Spannmittels mit dem Presselement, welche in die U-förmige Aufnahme der weiteren Knochenschraube eingesetzt sind.

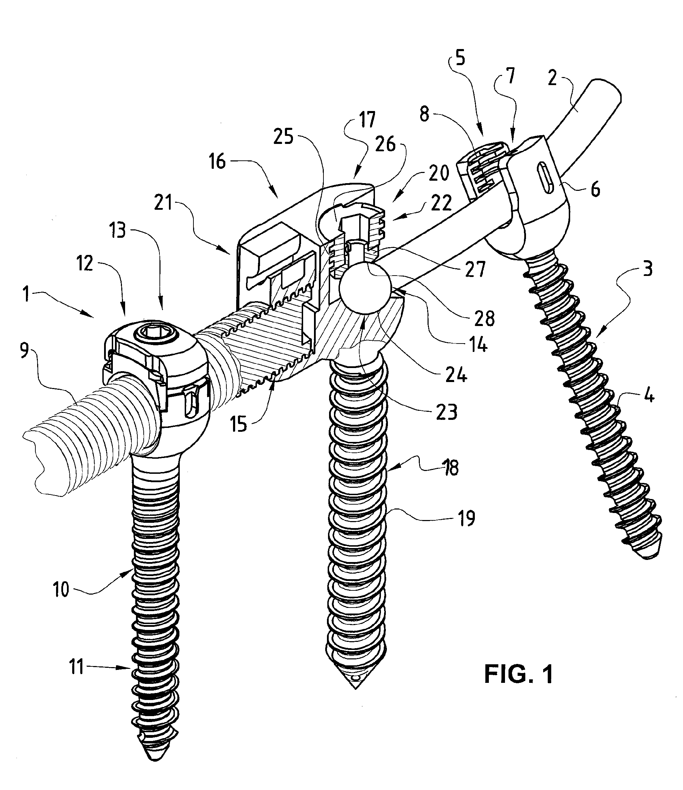

Das in Fig. 1 räumlich dargestellte Wirbelsäulenimplantat 1 weist ein erstes Verbindungselement 2 auf, das starr ist. Dieses erste Verbindungselement 2 ist in einer ersten Knochenschraube 3 gehalten, die in bekannter Weise aus einem Einschraubteil 4 besteht, welcher ebenfalls in bekannter, nicht dargestellter Weise in einen Wirbelkörper eingeschraubt werden kann. Zur Halterung des ersten Verbindungselementes 2 ist diese erste Knochenschraube 3 mit ersten Aufnahmemitteln 5 ausgestattet, die im Kopfteil 6 der ersten Knochenschraube 3 als U-förmige Aufnahme 7 ausgebildet sind. Das erste Verbindungselement 2, das als Stab ausgebildet ist, kann in diese U-förmige Aufnahme 7 eingelegt werden, die U-förmige Aufnahme 7 weist innenseitig ein Gewinde 8 auf, in welches in bekannter, nicht dargestellter Weise eine Spannschraube eingeschraubt werden kann, mittels welcher das erste Verbindungselement 2 in die U-förmige Aufnahme 7 eingespannt wird.

Das hier dargestellte Wirbelsäulenimplantat 1 umfasst ferner ein zweites Verbindungselement 9, das elastisch ist und das in einer zweiten Knochenschraube 10 gehalten ist. Diese zweite Knochenschraube 10 besteht ebenfalls aus einem Einschraubteil 11, welcher in bekannter, nicht dargestellter Weise in einen Wirbelkörper eingeschraubt werden kann. Im Kopfteil 12 dieser zweiten Knochenschraube 10 sind zweite Aufnahmemittel 13 angeordnet, in welchen das zweite Verbindungselement 9 in der zweiten Knochenschraube 10 fixiert und gehalten werden kann. Eine derartige zweite Knochenschraube mit den zweiten Aufnahmemitteln für die Aufnahme des zweiten Verbindungselementes ist in der europäischen Patentanmeldung mit der Veröffentlichungsnummer 2 074 957, auf welche hiermit verwiesen wird, im Detail beschrieben.

Wie aus Fig. 1 ersichtlich ist, ist der Endbereich 14 des ersten Verbindungselementes 2 und der Endbereich 15 des zweiten Verbindungselementes 9 in Verbindungsmitteln 16 verbindend gehalten. Diese Verbindungsmittel 16 sind im Kopfteil 17 einer weiteren Knochenschraube 18 angeordnet, welche weitere Knochenschraube 18 ebenfalls mit einem Einschraubteil 19 ausgestattet ist, mit welchem diese in bekannter, nicht dargestellter Weise ebenfalls in einen Wirbelkörper eingeschraubt werden kann.

Wie weiterhin aus Fig. 1 ersichtlich ist, umfassen die Verbindungsmittel 16, die im Kopfteil 17 der weiteren Knochenschraube 18 angebracht sind, eine erste Spanneinrichtung 20 zum Festhalten des ersten Verbindungselementes 2 und eine zweite Spanneinrichtung 21 zum Festhalten des zweiten Verbindungselementes 9, die später im Detail noch beschrieben wird. Die erste Spanneinrichtung 20 ist mit einer U-förmigen Aufnahme 22 ausgestattet, in welche der Endbereich 14 des ersten Verbindungselementes 2 eingesetzt werden kann. Der Endbereich 14 des ersten Verbindungselementes 2 ist kugelförmig ausgebildet, die Grundfläche 23 der U-förmigen Aufnahme 22 weist eine konkave Kugelform 24 auf, welche dem kugelförmigen Endbereich 14 des ersten Verbindungselementes 2 entspricht.

In der U-förmigen Aufnahme 22 ist ein Gewinde 25 angebracht, in welches eine Spannschraube 26 eingeschraubt werden kann. Beim hier dargestellten Ausführungsbeispiel ist zwischen der Spannschraube 26 und dem kugelförmigen Endbereich 14 des ersten Verbindungselementes 2, welches in die U-förmige Aufnahme 22 eingesetzt ist, ein Presselement 27 angeordnet. In bekannter Weise stützt sich die eine Oberfläche des Presselementes 27 auf der Spannschraube 26 ab, die dem kugelförmigen Endbereich 14 des ersten Verbindungselementes 2 zugewandte Oberfläche dieses Presselementes 27 weist eine konkave kugelförmige Ausnehmung 28 auf, die dem kugelförmigen Endbereich 14 des ersten Verbindungselementes 2 entspricht.

Beim in das Gewinde 25 eingeschraubten und angezogenen Zustand der Spannschraube 26 drückt diese somit auf das Presselement 27, die konkave kugelförmige Ausnehmung 28 wird auf den kugelförmigen Endbereich 14 des ersten Verbindungselementes 2 gepresst, dieser kugelförmige Endbereich 14 des ersten Verbindungselementes 2 wird dadurch wiederum auf die konkave Kugelform 24 der Grundfläche 23 der U-förmigen Aufnahme 22 gepresst, das erste Verbindungselement 2 wird dadurch in optimaler Weise in den Verbindungsmitteln 16 der weiteren Knochenschraube 18 gehalten. Durch die kugelförmige Ausgestaltung des Endbereichs 14 des ersten Verbindungselementes 2 und die entsprechenden konkaven kugelförmigen Aufnahmen 24 und 28 der ersten Spanneinrichtung 20 kann das erste Verbindungselement 2 vor dem Anziehen der Spannschraube 26 winklig ausgerichtet werden, so dass es in optimaler Weise bezüglich der ersten Knochenschraube 3 polyaxial angeglichen werden kann. Dadurch wird das Einsetzen eines ersten Verbindungselementes 2 in das vorgesehene Wirbelsäulenimplantat sehr vereinfacht, eine Ausrichtung durch Verbiegen des ersten Verbindungselementes 2 während des Einsetzens des Wirbelsäulenimplantats in eine zu stabilisierende Wirbelsäule ist dadurch nicht notwendig.

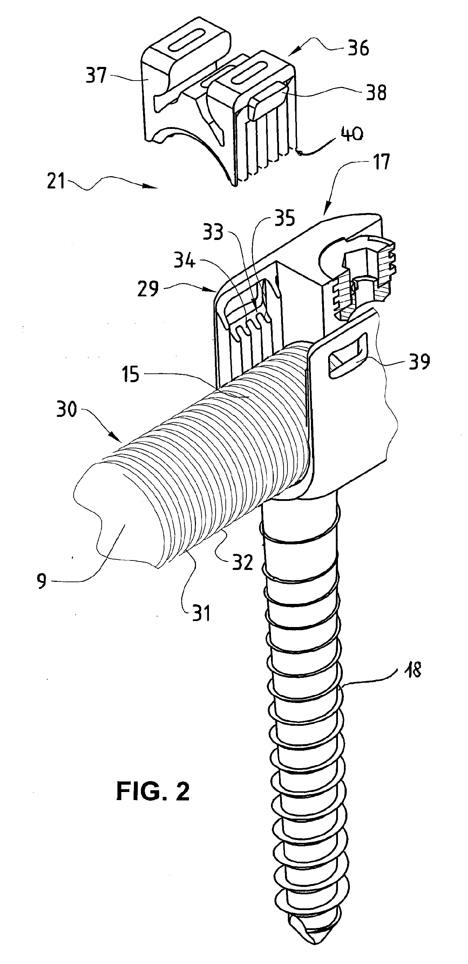

Wie aus Fig. 2 ersichtlich ist, weist die zweite Spanneinrichtung 21, die im Kopfteil 17 der weiteren Knochenschraube 18 angebracht ist, eine weitere U-förmige Aufnahme 29 auf. In diese weitere U-förmige Aufnahme 29 kann der Endbereich 15 des zweiten Verbindungselementes 9 eingesetzt werden. Mindestens dieser Endbereich 15 des zweiten Verbindungselementes 9 ist mit Strukturen 30 versehen, die im hier dargestellten Ausführungsbeispiel aus Rippen und Rillen 31, 32 ausgebildet sind, die im Wesentlichen quer zur Längsachse des zweiten Verbindungselementes 9 ausgerichtet sind. Wie aus Fig. 2 ersichtlich ist, ist die weitere U-förmige Aufnahme 29 mit weiteren Strukturen 33 versehen, die ebenfalls als Rippen 34 und Rillen 35 ausgebildet sind, welche den Rippen 31 und Rillen 32 des zweiten Verbindungsmittels 9 entsprechen. Durch diese Ausgestaltung erhält man zwischen der zweiten Spanneinrichtung 21 und dem Endbereich 15 des zweiten Verbindungselementes 9 eine formschlüssige Verbindung. Selbstverständlich wäre es auch denkbar, andere Strukturformen anzubringen, diese können beispielsweise nur in der weiteren U-förmigen Aufnahme 29 angebracht sein, die beim Einsetzen des Endbereiches 15 des zweiten Verbindungselementes 9 in diese weitere U-förmige Aufnahme 29 in deren Oberfläche eindringen, wodurch man ebenfalls eine praktisch formschlüssige Verbindung erhält.

Die weitere U-förmige Aufnahme 29 kann durch ein weiteres Spannmittel 36 verschlossen werden, welches im hier dargestellten Ausführungsbeispiel in bekannter Weise aus einem Einschiebeteil 37 gebildet ist, der aus einem elastischen Material besteht, und der mit Schnappelementen 38 ausgestattet ist, die im eingesetzten Zustand im in der weiteren U-förmigen Aufnahme 29 angebrachten Öffnungen 39 einschnappen können und den Einschiebeteil 37 in dieser Position halten. Der Einschiebeteil 37 kann ebenfalls mit Strukturen 40 versehen sein, die mit den Strukturen 30 des Endbereichs 15 des zweiten Verbindungselementes 9 bzw. den weiteren Strukturen 33 in der weiteren U-förmigen Aufnahme 29 zusammenwirken können.

Dieses weitere Spannmittel 36 könnte auch aus einer Spannschraube gebildet sein, die in ein entsprechend in der weiteren U-förmigen Aufnahme 29 angebrachtes Gewinde eingeschraubt werden könnte. Selbstverständlich wären auch andere bekannte und geeignete Spanneinrichtungen denkbar.

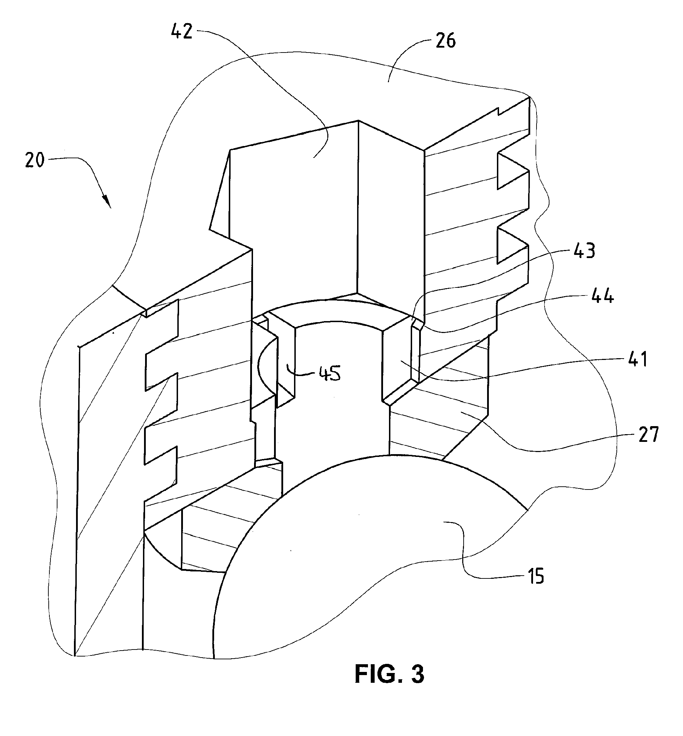

Wie aus der vergrösserten Darstellung gemäss Fig. 3 ersichtlich ist, kann die Spannschraube 26, welche in die erste Spanneinrichtung 20 (Fig. 1) eingeschraubt wird, und das eingesetzte Presselement 27 drehbar miteinander verbunden sein. Hierzu weist das Presselement 27 einen Vorsprung 41 auf, der in eine Öffnung 42 der Spannschraube 26 hineinragt, und der mit einer Rippe 43 ausgestattet ist. In der Öffnung 42 kann eine Nut 44 eingelassen sein, in welche die Rippe 43 eindringen kann. Der Vorsprung 41 ist mit Schlitzen 45 versehen, so dass dieser Vorsprung 41 in die Öffnung 42 eingepresst werden kann, wenn die Rippe 43 die Nut 44 erreicht, findet eine elastische Entspannung statt, das Presselement 27 ist somit mit der Spannschraube 26 drehbar verbunden, was eine vereinfachte Handhabung beim Einsetzen des Wirbelsäulenimplantats zur Folge hat. Die Rippe 43 und die Nut 44 können mit Spiel versehen sein, so dass hier beim Spannen der Spannschraube 26 und des Anpressen des Presselementes 27 keine Kräfte auftreten.

Das in einem derartigen Wirbelsäulenimplantat eingesetzte elastische zweite Verbindungsmittel besteht in vorteilhafter Weise aus einem biokompatiblen Kunststoff auf Polyurethanbasis, während die anderen Teile aus einer Titanlegierung bestehen. Selbstverständlich wären auch andere geeignete Materialien verwendbar.

Vorgängig ist ein Ausführungsbeispiel beschrieben worden, das jeweils insgesamt aus drei Schrauben gebildet ist, wobei die erste und mittlere Schraube über ein starres Verbindungselement miteinander verbunden sind, und die mittlere Schraube und die zweite Schraube über ein elastisches Verbindungselement miteinander verbunden sind. Selbstverständlich sind auch viele andere Ausführungsbeispiele denkbar, die mehr Schrauben aufweisen, bei welchen die starren und elastischen Verbindungselemente sich beispielsweise über mehr als zwei Schrauben erstrecken, es ist auch denkbar, dass mehr als zwei Verbindungselementteile verwendet werden, beispielsweise kann zwischen zwei elastischen Verbindungselementen ein starres Verbindungselement eingesetzt werden, wobei im Bereich der Verbindung dieser Verbindungselemente die vorgängig beschriebenen weiteren Knochenschrauben einzusetzen wären. Je nach Bedarf kann somit ein praktisch beliebig zusammengestelltes Wirbelsäulenimplantat verwendet werden.

Mit dieser Erfindung wird ein Wirbelsäulenimplantat zur Stabilisierung und Versteifung von Wirbelkörpern einer Wirbelsäule erhalten, die in optimaler Weise an die Begebenheiten angepasst werden kann, welche in optimaler Weise eine polyaxiale Anpassung eines starren Verbindungselementes bezüglich eines elastischen Verbindungselementes ermöglicht, und welche einfach ist in der Handhabung.

## Patentansprüche

1. Wirbelsäulenimplantat zur Stabilisierung und Versteifung von Wirbelkörpern einer Wirbelsäule, umfassend mindestens eine erste Knochenschraube (3), bestehend aus einem Einschraubteil (4), welcher in einen Wirbelkörper einschraubbar ist, und ersten Aufnahmemitteln (5), mindestens eine zweite Knochenschraube (10), bestehend aus einem Einschraubteil (11), welcher in einen Wirbelkörper einschraubbar ist, und zweiten Aufnahmemitteln (13), mindestens ein erstes Verbindungselement (2), das starr ist und in die ersten Aufnahmemittel (5) der ersten Knochenschraube (3) einsetzbar und darin befestigbar ist, mindestens ein zweites Verbindungselement (9), das elastisch ist und in die zweiten Aufnahmemittel (13) der zweiten Knochenschraube (10) einsetzbar und darin befestigbar ist, und Verbindungsmittel (16), mit welchen jeweils ein erstes Verbindungselement (2) mit einem zweiten Verbindungselement (9) verbindbar ist, **dadurch gekennzeichnet, dass** die Verbindungsmittel (16) an einer weiteren Knochenschraube (18) angebracht sind und aus einer ersten Spanneinrichtung (20) zum Festhalten des ersten Verbindungselementes (2) und aus einer zweiten Spanneinrichtung (21) zum Festhalten des zweiten Verbindungselements (9) bestehen, und dass die erste Spanneinrichtung (20) eine U-förmige Aufnahme (22) für den Endbereich (14) des ersten Verbindungselementes (2) aufweist, welcher Endbereich (14) kugelförmig ausgebildet ist, und auf die U-förmige Aufnahme (22) ein Spannmittel (26, 27) aufsetzbar ist, mit welchem der kugelförmige Endbereich (14) gegen die Grundfläche (23) der U-förmigen Aufnahme (22) spannbar ist.

2. Wirbelsäulenimplantat nach Anspruch 1, **dadurch gekennzeichnet, dass** die Grundfläche (23) der U-förmigen Aufnahme (22) eine konkave Kugelform (24) aufweist, die dem kugelförmigen Endbereich (14) des ersten Verbindungselementes (2) entspricht.

3. Wirbelsäulenimplantat nach Anspruch 1 oder 2, **dadurch gekennzeichnet, dass** die dem kugelförmigen Endbereich (14) des ersten Verbindungselementes (2) zugewandte Oberfläche des Spannmittels (26, 27) eine konkave kugelförmige Ausnehmung (28) aufweist.

4. Wirbelsäulenimplantat nach einem der Ansprüche 1 bis 3,
**dadurch gekennzeichnet, dass** das Spannmittel eine Spannschraube (26) umfasst, welche in ein in der U-förmigen Aufnahme (22) angebrachtes Gewinde (25) einschraubbar ist.

5. Wirbelsäulenimplantat nach Anspruch 4, **dadurch gekennzeichnet, dass** das Spannmittel ein Presselement (27) umfasst, welches zwischen die Spannschraube (26) und den kugelförmigen Endbereich (14) des ersten Verbindungselementes (2) einsetzbar ist.

6. Wirbelsäulenimplantat nach Anspruch 5, **dadurch gekennzeichnet, dass** die Spannschraube (26) drehbar mit dem Presselement (27) verbunden ist.

7. Wirbelsäulenimplantat nach einem der Ansprüche 1 bis 6,
**dadurch gekennzeichnet, dass** die zweite Spanneinrichtung (21) eine weitere U-förmige Aufnahme (29) für den Endbereich (15) des zweiten Verbindungselementes (9) aufweist, auf welche ein weiteres Spannmittel (36) aufsetzbar ist, mit welchem der Endbereich (15) des zweiten Verbindungselementes (9) in der weiteren U-förmigen Aufnahme (29) gehalten ist.

8. Wirbelsäulenimplantat nach Anspruch 7, **dadurch gekennzeichnet, dass** die dem Endbereich (15) des zweiten Verbindungselements (9) zugewandte Fläche des weiteren Spannmittels (36) und/oder die weitere U-förmigen Aufnahme (29) mit Strukturen (30) versehen sind.

9. Wirbelsäulenimplantat nach einem der Ansprüche 7 oder 8,
**dadurch gekennzeichnet, dass** mindestens der Endbereich (15) des zweiten Verbindungselementes (9) mit weiteren Strukturen (33) versehen ist, die zu den Strukturen (30) der weiteren U-förmigen Aufnahme (29) komplementär ausgebildet sind.

10. Wirbelsäulenimplantat nach einem der Ansprüche 8 oder 9,
**dadurch gekennzeichnet, dass** die Strukturen (30) und die weiteren Strukturen (33) aus Rippen und Rillen (31,32; 34,35) bestehen, die im Wesentlichen quer zur Längsachse des zweiten Verbindungselementes (9) ausgerichtet sind.
